(19) Europäisches Patentamt
European Patent Office
Office européen des brevets

(11) Publication number: **0 190 815 B1**

(12) **EUROPEAN PATENT SPECIFICATION**

(45) Date of publication of patent specification: **02.05.90**

(21) Application number: **86300041.0**

(22) Date of filing: **06.01.86**

(51) Int. Cl.[5]: **C 07 C 39/08**, C 07 C 37/01, C 07 C 43/23, C 07 C 41/18, C 07 C 37/055, C 07 C 67/03, C 07 C 67/42, C 07 C 69/157

(54) **Process for producing aromatic diols.**

(30) Priority: **07.01.85 US 689533**

(43) Date of publication of application:
**13.08.86 Bulletin 86/33**

(45) Publication of the grant of the patent:
**02.05.90 Bulletin 90/18**

(84) Designated Contracting States:
**AT BE CH DE FR GB IT LI LU NL SE**

(56) References cited:
**EP-A-0 178 929**

**JOURNAL OF THE AMERICAN CHEMICAL SOCIETY, vol. 72, no. 12, 2nd January 1951, pages 5515-5518, Columbus, Ohio, US; W. VON E. DOERING et al.: "The peracetic acid cleavage of unsymmetrical ketones"**

**CHEMICAL ABSTRACTS, vol. 93, no. 1, 7th July 1980, page 711, column 1, abstract no. 7777n, Columbus, Ohio, US; N. HIRAO et al.: "Synthesis of dihydroxybenzene by oxidation of hydroxyacetophenone", & KOEN YOSHISHU - KORYO, TERUPEN OYOBI SEIYU KAGAKU NI KANSURU TORONKAI, 23Rd 1979, 131-133**

(73) Proprietor: **CELANESE CORPORATION**
**1211 Avenue of the Americas**
**New York New York 10036 (US)**

(72) Inventor: **Hilton, Charles Bruce**
**505 Williamson Place**
**Corpus Christi Texas (US)**

(74) Representative: **De Minvielle-Devaux, Ian Benedict Peter et al**
**CARPMAELS & RANSFORD 43, Bloomsbury Square**
**London WC1A 2RA (GB)**

**EP 0 190 815 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to an improved process for the production of aromatic diols.

Background of the Invention

Aromatic diols such as hydroquinone are known commodities of commerce having use, for example as photographic developers, polymerization inhibitors, dye intermediates and anti-oxidants. Hydroquinone (HQ) has been commercially produced by the manganese oxide-catalyzed oxidation of aniline to quinone followed by the iron-catalyzed reduction of the quinone to hydroquinone. A more recently developed method of producing hydroquinone comprises the diisopropylation of benzene to form para-diisopropylbenzene followed by peroxidation of the latter compund and acid-catalyzed decompsition of the diperoxide to produce hydroquinone and acetone. While there are advantages to each of these processes, a process of making aromatic diols such as hydroquinone with a lower cost of construction of a commercial unit and/or a lower cost of production would be highly desirable.

The monoalkyl ethers of aromatic diols are also known compounds. In addition to some of the same uses mentioned for aromatic diols, these compounds have use as bactericides and antiseptics, pharmaceuticals e.g. as expectorants and depigmentors, and fragrances.

Aromatic diols and their dicarboxylate esters, e.g. the diacetate ester of hydroquinone (HQ), have also found application as monomers in the preparation of certain polymers, e.g. those capable of forming an anisotropic melt phase and suitable for being formed into shaped articles such as molded articles, fibers and films as shown, for example in U.S. Patents Nos. 4,330,457; 4,351,918; and 4,355,132.

With regard to the process disclosed and claimed herein, the oxidation of ketones with peroxy compounds to form esters, i.e. the "Baeyer-Villiger" reaction, is known in the art. Some references with disclosures of examples of this type of reaction and descriptions of their teachings are as follows:

Hirao et al, Koen Yoshishu-Koryo, Terupen oyobi Seiyu Kagaku ni kansuru Toronkai, 23rd, 1979, 131—3 (Japan). Chem. Soc. Japan: Tokyo, Japan, as abstracted in C.A. (1980,) *93*, 7777n, show the Fries rearrangement of phenyl acetate and the Friedel-Crafts acetylation of phenol to form para-hydroxy-acetophenone which is then oxidized with hydrogen peroxide using sulfuric acid as catalyst to para-hydroxyphenyl acetate; the latter is then hydrolyzed with sulfuric acid in acetic acid to yield hydroquinone.

Von E. Doering et al, Journal of the American Chemical Society *72*, 5515—5518 (1950) teach the Baeyer-Villiger oxidation of various aromatic ketones, including acetophenone and para-substituted benzophenones, to esters using peracetic acid as the oxidant and show that sulfuric acid has a "marked catalytic action" with regard to the yield and reaction time (page 5517, col. 1).

Ogata et al, Journal of Organic Chemistry *43*, No. 12, 2417—2419 teach the Baeyer-Villiger oxidation of acetophenone and substituted acetophenones to the corresponding aryl acetates using permonophosphoric acid as oxidant and sulfuric acid as catalyst and show that the reaction rate is highly dependent on the acidity of the solution.

Starcher et al, Journal of the American Chemical Society, *80*, 4079—4082 (1958) teach the synthesis of lactones by means of the Baeyer-Villiger oxidation of cyclic ketones using peracetic acid as oxidant. In the paragraph bridging pages 4079 and 4080, the authors state: "The absence of inorganic impurities, notably water, hydrogen peroxide, mineral acids and salts, reduced polymerization to a minimum during the reaction step and avoided many of the by-products which plagued previous investigators."

Adam et al, Journal of Organic Chemistry, *44*, No. 26, 4969 (1979) describe the use of bis(trimethylsilyl) monoperoxysulfate as a Baeyer-Villiger oxidant in the oxidation of ketones to their corresponding esters. The authors state that the shortcomings of a previously-used oxidant for this purpose, Caro's or monoperoxysulfuric acid, are "(i) the use of aqueous conditions, (ii) the presence of strong acid and (iii) undesirable side reactions."

The alcoholysis or transesterification of an ester by reaction with an alcohol to form another ester of the acid moiety of the first ester with the alcolyzing alcohol, and the free alcohol of the first ester, is also known in the art. Thus, Head, U.S. Patent No. 2,639, 298 shows the "deesterification" of saligenin diacetate or substituted saligenin diacetates with methanol to produce saligenin or a substituted saligenin and methyl acetate.

Fieser and Fieser "Advanced Organic Chemistry", Reinhold (1961), page 378, show the reaction of trilaurin with methanol to form methyl laurate and glycerol.

Solomons, "Organic Chemistry, Second Edition" Wiley (1980), page 771 shows the reaction of methyl acrylate with n-butyl alcohol to form n-butyl acrylate and methyl alcohol.

EP—A—178929 based on USSN 661,552 filed October 17, 1984 discloses and claims a method of producing aromatic diols or monomethyl ethers of aromatic diols by first subjecting an aromatic ketone containing a hydroxy, alkoxy or acyloxy radical on the aromatic ring, e.g. 4-hydroxyacetophenone, to a Baeyer-Villiger oxidation using peracetic acid as oxidant under conditions such that the reaction mass contains no more than about 0.1 wt% of sulfuric acid based on the weight of the initially added pure peracetic acid. The carboxylate esters may be hydrolyzed to the aromatic diols, e.g. hydroquinone, or to the monoalkyl ethers of the aromatic diols. e.g. the monomethyl ether of hydroquinone, by reacting the ester with water under acid conditions.

While the recovery of the aromatic diol or the monoalkyl ether of the aromatic diol from the

corresponding monocarboxylate ester as disclosed in previously cited application Serial N. 661,552 and the cited Hiroa et al article is satisfactory for many purposes, such method of recovery may have some disadvantages under certain circumstances. Thus, the hydrolysis of the monocarboxylate ester results in the formation of a free carboxylic acid as a by-product, e.g. acetic acid when a monoacetate is hydrolyzed. Although such carboxylic acid by-product may have value, it is obtained, due to the amount of water used for the hydrolysis reaction, as a relatively dilute aqueous solution from which it is uneconomical to recover the acid. Furthermore, the separation of carboxylic acid by-product from the aromatic diol or monoalkyl ether of an aromatic diol product may be more difficult and consume a larger amount of energy than other by-products obtained from alternative recovery processes.

Summary of the Invention

In accordance with this invention, a composition comprising a major proportion of a carboxylate ester of an aromatic diol, e.g., the monoacetate of hydroquinone (MAHQ), is reacted with an alcohol or phenol in an transesterification reaction to form an aromatic diol, e.g. hydroquinone (HQ) (hereinafter referred to as the "product phenolic compound") and a relatively easy to recover ester of the acid moiety of the aforesaid carboxylate ester and the reacting alcohol or phenol (hereinafter referred to as the "product ester"). The foregoing reaction may also be described as an "alcoholysis" if an alcohol is initially reacted with the ester of an aromatic diol or a "phenolysis" if a phenol is initially reacted.

The composition comprising the initial ester is produced by subjecting an aromatic ketone, containing a hydroxy radical on the aromatic ring, e.g. 4-hydroxy-acetophenone (4—HAP), to a "Baeyer-Villiger" oxidation using as oxidant peracetic acid. The oxidation is carried out using the process disclosed in previously cited EP—A—178929 including the use as oxidant of peracetic acid under conditions such that the reaction mass contains no more than 0.1 weight percent of sulfuric acid based on the weight of the initially added pure peracetic acid. Desirably, the reaction mass also contains no more than 5 weight percent of water based on the weight of pure peracetic acid initially added. The procedure excludes the use in unmodified form of most commercial peracetic acid solutions which generally contain sulfuric acid and water in amounts greater than the maximum amounts previously set out.

The transesterification reaction of this invention proceeds as in equation (I):

$$YO—Ar^1—O\overset{\overset{O}{\|}}{C}R + R^1OH \xrightarrow{\text{acid}} YO—Ar^1—OH + R^1O\overset{\overset{O}{\|}}{C}R \quad (I)$$

where R is an alkyl radical, $R^1$ is an alkyl, aryl or aralkyl radical, $Ar^1$ is a divalent aromatic radical (all more specifically defined below) and Y is hydrogen, or RC = O. If Y is RC = O, i.e. the initial aromatic diol ester is a diester, then the monoester as well as the diol will form if an insufficient amount of alcohol or phenol is supplied to the reaction. Thus, in this case, a sufficient amount of alcohol or phenol must be supplied to react with two carboxylate radicals per molecule of initial aromatic diol ester.

If it is desired to transesterify the monoacetate of hydroquinone (MAHQ) to hydroquinone (HQ) and ethyl acetate using ethanol as the alcoholyzing alcohol, the reaction proceeds as in equation (II):

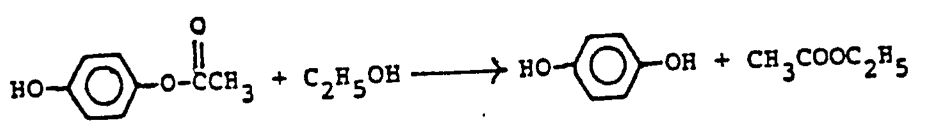
(II)

The carboxylate esters of aromatic diols contemplated to be transesterified to aromatic diols in accordance with this invention, may be produced by the Baeyer-Villiger oxidation of the corresponding aromatic ketone as shown in equation (III):

$$YO—Ar^1—\overset{\overset{O}{\|}}{C}R + [O] \xrightarrow[\text{compound}]{\text{peroxy}} YO—Ar^1—O\overset{\overset{O}{\|}}{C}R \quad (III)$$

If it is desired to produce MAHQ from 4-hydroxyacetophenone (4-HAP) by a Baeyer-Villiger oxidation using paracetic acid as oxidant, the reaction proceeds as in equation (IV):

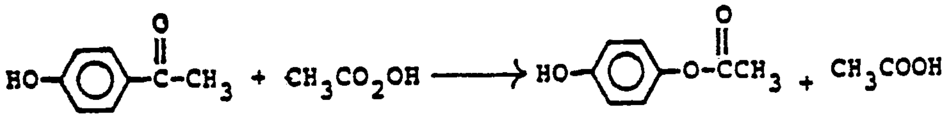 (IV)

In equations (1) and (II) $Ar^1$ is a divalent aromatic radical. The specific nature of the radical is not critical but it is preferably a radical resulting from the removal of two ring hydrogen atoms from benzene, naphthalene, or biphenyl, either unsubstituted or with ring hydrogens substituted with radicals such as methyl, tolyl; and appropriately masked amino, or sulfhydryl substituents. $Ar^1$ is preferably 1,4-phenylene, 2,1-naphthylene, 2,6-naphthylene, 5-phenyl-1,2-phenylene, 3-phenyl-1,4-phenylene or 3-methyl-1,4-phenylene with the ketocarbon and corresponding groups occupying the first stated numbered position of $Ar^1$ when the positions are not equivalent. Most preferably $Ar^1$ is 1,4-phenylene.

The R groups in equations (I) and (III) are alkyl groups containing, for example 1 to 18 carbon atoms, preferably 1 to 4 carbon atoms. More preferably, R is methyl, ethyl or propyl and most preferably methyl corresponding to the use of methyl ketones and ethers and acetate esters in the latter equations.

The $R^1$ radical in equation (I) may be alkyl containing 1 to 18 carbon atoms, preferably 1 to 4 carbon atoms; aryl such as phenyl or naphthyl which can be unsubstituted or substituted with radicals inert under the conditions of reaction such as alkyl, aryl, alkoxy or chloride: or aralkyl, e.g. having the formula:

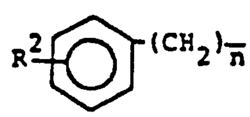

where $R^2$ may be hydrogen or one or more inert substituents, e.g. alkyl, aryl, alkoxy or chloride, and n may be, for example, in the range of 1 to 4. The foregoing alkyl and alkoxy substituents may have a number or carbon atoms within the same ranges specified previously for R and $R^1$ when $R^1$ is alkyl. In particularly preferred embodiments $R^1$ is ethyl or n-butyl corresponding to the use of ethanol or n-butanol as the alcoholyzing alcohol. Most preferably the starting material for the transesterification is the monoacetate ester of hydroquinone (MAHQ) formed by the Baeyer-Villiger oxidation of 4-hydroxyacetophenone (4-HAP) and the products of the reaction are hydroquinone (HQ) and ethyl acetate formed by the alcoholysis of the MAHQ with ethanol.

In producing esters of aromatic diols in accordance with equation (I), the hydroxy aromatic keton is subjected to a Baeyer-Villiger oxidation by reacting the ketone with the peroxy compound peracetic acid as indicated in the claims.

The amount of peroxy compound supplied to the reaction is in the range for example of about 0.5 to about 2 moles per mole of aromatic ketone. The reaction may be carried out at a temperature, for example, of about 20 to 140°C and a pressure, for example, of about 25 mm of mercury to about 2 atmospheres for a period, for example, of about 0.5 to about 3 hours. A solvent for the aromatic ketone may be used in the initial reaction solution e.g. an alkanoic acid such as glacial acetic acid or formic acid.

Desirably, the Baeyer-Villiger oxidant is a purified peracetic acid which is contacted with the aromatic ketone under such conditions that the reaction solution contains no more than 0.1 wt.%, preferably no more than about 0.05 wt.% of sulfuric acid based on the weight of pure peracetic acid initially added. Advantageously the reaction solution also contains no more than 5 wt.%, preferably no more than about 2 wt.% of water based on the weight of pure peracetic acid. In general, the less the sulfuric acid and water present the better. Preferably, the peracetic acid is added in the form of a solution consisting essentially of about 5 to 45 wt% peracetic acid and the remainder a solvent such as acetic acid, methyl acetate, ethyl acetate, acetone or mixtures thereof.

The hydroxy aromatic ketones which are starting materials for the production of monocarboxylate esters of aromatic diols are compounds known in the art and may be produced by any known process. Preferably, however, they are produced by the Fries rearrangement of aryl carboxylates such as phenyl acetate, or the Friedel-Crafts acylatioon of phenols, e.g. the acetylation of phenol with acetic acid or anhydride, both reactions using hydrogen fluoride as catalyst, as disclosed, for example in previously cited copending application Serial No. 661.552.

The transesterification of the monocarboxylate ester of the aromatic diol is carried out by contacting the ester with the alcoholyzing alcohol or phenolyzing phenol under acidic conditions and elevated temperature for a period of time sufficient to permit the reaction to approach completion. Typically the amount of alcohol used will be for example about 50 to 1000 preferably about 100 to 200 mol% based on the ester being alcoholyzed, the temperature will be about 20 to 200°C (broad range), preferably about 60 to 140°C and the period of reaction will be for example, about 0.1 to 10, preferably about 0.5 to 4 hours. A solvent may be employed which may be, for example, the product ester or the acid moiety of the initial and product esters in free form, alone or in admixture, e.g. ethyl acetate and/or acetic acid in the case of the alcoholysis of the monoacetate of hydroquinone (MAHQ) with ethanol. Other solvents which may be used are n-butyl acetate, propyl acetate, butyric acid, and propionic acid. However, use of these solvents in the alcoholysis of MAHQ with ethanol would complicate the solvent recovery system. If a solvent is employed,

it is used in such quantity as to yield a solution of about 5 to 60 wt%, preferably about 25 to 40 wt.% of monocarboxylate ester of either the aromatic diol or monoalkyl.ether of an aromatic diol. Stirring or other mixing action is preferably employed during the reaction.

The reaction is carried out in the presence of an acid catalyst. Acids which may be employed for this purpose are organic acids such as methanesulfonic acid, para-toluenesulfonic acid and benzenesulfonic acids, mineral acids such as sulfuric, hydrochloric and phosphoric acids and acidic ion-exchange resins e.g. sulfonic acid ion-exchange resins such as Amberlyst 15 sold by Rohm and Haas and Nafion 501 sold by duPont. The acid is usually employed in an amount, for example of about 0.1 to 5, preferably about 0.5 to 2 mol% based on the monocarboxylate ester.

In some instances, it may be desirable to employ a combination of alcohol or phenol and water to achieve a measure of both transesterification and hydrolysis of the monocarboxylate ester to obtain the aromatic diol or monoalkyl ether of aromatic diol and a mixture of newly synthesized ester and esterifying acid. For example, there may be some advantage to having a small amount of water remaining after the reaction since this may facilitate separation of an alcoholyzing alcohol, e.g. ethanol, and the product ester, e.g. ethyl acetate due to a favorable phase separation. If a mixture of alcohol or phenol and water is employed, the water will usually be present in an amount, for example, of about 25 to 100 mol% based on the alcohol. In this case, the combination of water and alcohol will generally be used in an amount within the ranges given above for the amount of alcohol employed when it is used without water. The product may be recovered by conventional purification methods usually involving a combination of crystallization, filtration, washing and distillation in any order deemed advantageous for the system at hand.

The transesterification process of this invention provides for the recovery of product phenolic compound in relatively high yields. Thus the process generally results in a conversion of initial ester to all products of at least about 95%, preferably at least about 98% with efficiency to product phenolic compound of at least about 97%, preferably at about 99% and an efficiency to product ester of at least about 97%, preferably at least about 99%.

Description of Specific Embodiments

The following examples further illustrate the invention.

Examples 1 to 7 illustrate the conversion of 4-hydroxy-acetophenone (4-HAP) to the monoacetate ester of hydroquinone (MAHQ) by the Baeyer-Villiger oxidation using a "purified" peracetic acid containing under 0.05 wt% of sulfuric acid and under 2 wt.% of water as the oxidizing agent.

Example 1

To 650 grams of commercially available peracetic acid were slowly added 650 grams of acetic anhydride. This commercial acid contained ~40 wt.% peracetic acid, ~13 wt.% water, ~5 wt.% hydrogen peroxide and ~1 wt.% sulfuric acid, and the purpose of the acetic anhydride treatment was to convert the water and hydrogen peroxide present to acetic acid and peracetic acid, respectively. The solution was cooled and stirred during the addition to maintain the temperature at <30°C. The solution was then distilled under vacuum (20—30 mmHgA, ~40°C) through a one-inch Vigreux column. The initial 20 cc. of distillate were discarded. Then, ~1000cc. of material was distilled without reflux. This material referred to below as "purified peracetic acid solution" contained ~27 wt.% peracetic acid and ~73 wt.% acetic acid and was used for the oxidations of this and the following examples.

In a solution of 60 grams of glacial acetic acid and 10 grams of acetic anhydride were dissolved 30 grams of 4-HAP. This solution was placed into a 250 cc. three-neck flask. While heating the solution to 60°C, the flask was partially evacuated to a pressure of 60 mmHgA. This pressure was maintained throughout the experiment. To the solution were added 77.5 grams of the purified peracetic acid solution containing 20.46 g of pure peracetic acid, fed dropwise over a period of 40 minutes, and the solution was heated for an additional 80 minutes. The initial mole ratio of peracetic acid to 4-HAP was 1.22. The solution was then cooled and analyzed by gas chromatography and other well-established procedures.

The product solution weighed 171.3 grams and contained 18.0 wt.% monoacetate ester of hydroquinone (MAHQ), 0.47 wt.% diacetate ester of hydroquinone (DAHQ), 0.03 wt.% hydroquinone (HQ), 0.05 wt.% benzoquinone (BQ), 0.17 wt.% 4-acetoxyacetophenone (4-AAP), and 0.41 wt.% of 4-hydroxyacetophenone (4-HAP). Peracetic acid was not detected. This corresponds to 97% conversion of 4-HAP and 97% efficiency to hydroquinone and its esters.

Example 2

The procedure of Example 1 was followed except that the temperature employed was 80°C, the solvent consisted of 70 g acetic acid and no acetic anhydride to which 4.4 mg of ethylene diamine tetraacetic acid (EDTA) were added and 51.7 grams of purified peracetic acid solution containing 13.65 of pure peracetic acid were fed over the 40 min. period. The initial mole ratio of peracetic acid to 4-HAP was 0.81.

The product solution weighed 154.0 grams and contained 13.7 wt.% MAHQ, 0.33 wt.% DAHQ, 0.78 wt.% HQ, 0.03 wt.% BQ, and 4.8 wt.% of 4-HAP, corresponding to 74.93% conversion of 4-HAP and 92.17% efficiency to HQ and its esters.

Example 3

The procedure of Example 1 was followed except that the solvent consisted of 70 grams of acetic and no acetic anhydride to which 4.4 mg. of EDTA were added, and the purified peracetic acid solution was added over a 10 min. period and the reaction solution heated for an additional 40 min. period. The initial mole ratio of peracetic acid to 4-HAP was 1.22.

The product solution weighed 177.3 grams and contained 16.8 wt.% MAHQ, 0.26 wt.% of DAHQ, 0.13 wt.% HQ, 0.13 wt.% BQ and 0.02 wt.% 4-HAP, corresponding to 99.88% conversion of 4-HAP and 90.97% efficiency to HQ and its esters.

Example 4

The procedure of Example 2 was followed except that 51.7 grams of purified peracetic acid solution containing 14.37 grams of peracetic acid were fed over a 20 min. period, the solution was heated for an additional 40 min. and the solvent consisted of 60 grams of acetic acid and 10 grams of acetic anhydride. The initial mole ratio of peracetic acid to 4-HAP was 0.86.

The product solution weighed 153.0 grams and contained 14.10 wt.% MAHQ, 1.10 wt.% DAHQ, 0.77 wt.% HQ, 0.08 wt.% BQ and 4.60 wt.% 4-HAP corresponding to 75.41% conversion of 4-HAP and 97.97% efficiency to HQ and its esters.

Example 5

The procedure of Example 1 was followed except that 62.1 grams of purified peracetic acid solution containing 17.26 grams of peracetic acid were fed over a 30 min. period, the solution was heated for an additional 30 min. and the solvent consisted of 70 grams of acetic acid to which was added 0.1 gram of sodium acetate. The initial mole ratio of peracetic acid to 4-HAP was 1.03.

The product solution weighed 161.7 grams and contained 21.40 wt.% MAHQ, 0.00 wt.% DAHQ, 0.02 wt.% HQ, 0.00 wt.% BQ and 0.55 wt.% 4-HAP corresponding to a conversion of 4-HAP of 97.04% and an efficiency to HQ and its esters of 100%.

Example 6

The procedure of Example 1 was followed except that 107.4 grams of purified peracetic acid solution containing 29.11 grams of peracetic acid were fed over a 30 min. period, the solution was heated for an additional 30 min. and the 4-HAP solution consisted of 50.8 grams of acetic acid and a 50.8 grams of 4-HAP. The initial mole ratio of peracetic acid to 4-HAP was 1.04.

The product solution weighed 206.9 grams and contained 21.9 wt.% MAHQ, 0.09 wt.% DAHQ, 0.01 wt.% BQ, 0.01 wt.% HQ and 1.70 wt.% 4-HAP corresponding to a normalized conversion of 4-HAP based on 88.85% accountability of products of 91.69% and a normalized efficiency to HQ and its esters of 99.9%.

Example 7

The procedure of Example 1 was followed except that 90.8 grams of purified peracetic acid solution containing 24.61 grams of peracetic acid were fed over a 30 min. period, the solution was heated for an additional 30 min. and the 4-HAP solution consisted of 69.1 grams of 90% formic acid and 40.3 grams of 4-HAP. The initial mole ratio of peracetic acid to 4-HAP was 1.09.

The product solution weighed 198.5 grams and contained 17.6 wt.% MAHQ, 0.17 wt.% DAHQ, 0.85 wt.% HQ, 0.04 wt.% BQ and 1.10 wt.% 4-HAP corresponding to a normalized conversion of 4-HAP based on 88.99% accountability of products of 93.91% and a normalized efficiency to HQ and its esters of 99.70%.

Example 8 (For Comparison)

This example illustrates the use of hydrogen peroxide as the Baeyer-Villiger oxidant.

The procedure of Example 7 was followed except that 26.1 grams of a 50 wt.% hydrogen peroxide solution in water were used as the oxidant in place of the peracetic acid solution and the 4-HAP solution consisted of 53.2 grams of 90% formic acid and 30.0 grams of 4-HAP. The initial mole ratio of hydrogen peroxide to 4-HAP was 1.09.

The product solution weighed 105.2 grams and contained 6.00 wt.% MAHQ, 0.0 wt.% DAHQ, 8.10 wt.% HQ, 0.0 wt.% BQ and 5.00 wt.% 4-HAP corresponding to a normalized conversion of 4-HAP of 75.47% and a normalized efficiency to HQ and its esters of 100.00% based on 71.48% accountability of products.

Comparison of the results of Example 8 with those of Example 7 indicates that the use of peracetic acid in the absence of sulfuric acid as oxidant yields higher conversions of 4-HAP than hydrogen peroxide with equivalent efficiencies to HQ and its esters. This indicates that the use of such peracetic acid will result in somewhat higher transesterification yields of aromatic diol than if other peroxy compounds such as hydrogen peroxide is used as the Baeyer-Villiger peroxy oxidant.

Example 9

This example illustrates the alcoholysis of MAHQ and DAHQ with ethanol to form HQ and ethyl acetate.

Product from several experiments as described in Example 1 were combined and most of the acetic acid removed by evaporation under vacuum at 100°C. Analysis of the non-volatile portion showed that it contained 81.5 wt.% MAHQ, 2.0 wt.% HQ, 2.2 wt.% DAHQ, 1.5 wt.% 4-HAP, 0.01 wt.% BQ, and 1.2 wt.%

acetic acid. 28.0 Grams of this non-volatile material were added to a solution containing 25.9 grams of ethyl acetate, 11.0 grams of ethanol, 13.0 grams of acetic acid, and 0.29 gram of 70% methanesulfonic acid. This solution was stirred and refluxed at 87°C for two hours. Upon cooling to 0°C a crystalline solid precipitated. The solid was filtered, washed with 10 ml of ethyl acetate, and dried in a vacuum dessicator. The dried solid weighed 2.59 grams and contained 93.9 wt.% HQ, 0.06 wt.% MAHQ, 0.0 wt.% DAHQ, 0.0 wt.% BQ, and 1.2 wt.% acetic acid. The filtrate plus wash weighed 75.8 grams and contained 23.5 wt.% HQ, 0.30 wt.% MAHQ, 0.0 wt.% DAHQ, 13.2 wt.% acetic acid, 63.3 wt.% ethyl acetate (an increase of 22.1 grams) and 2.1 wt.% ethanol (a decrease of 9.4 grams). These results show that the normalized conversion of MAHQ to HQ was 99.1% and that normalized efficiency of HQ was 100% based on product accountability of 117.1%.

Example 10

This example illustrates the combined hydrolysis and alcoholysis of MAHQ and DAHQ with water and ethanol to form HQ and ethyl acetate.

Product from several experiments as described in Example 1 were combined and most of the acetic acid removed by evaporation under vacuum at 100°C. Analysis of the non-volatile portion showed that it contained 81.5 wt.% MAHQ, 2.0 wt.% BQ, 2.2 wt.% DAHQ, 1.5 wt.% 4-HAP, 0.01 wt.% BQ, and 1.2 wt.% acetic acid. 30.0 Grams of this non-volatile material were added to a solution containing 25.5 grams of ethyl acetate, 7.9 grams of ethanol, 12.0 grams of acetic acid, 3.1 grams of water and 0.14 gram of 70% methanesulfonic acid. This solution was stirred and refluxed at 87.6°C for one hour. The product was cooled to 0°C, but no precipitate formed. The product weighed 77.5 grams and contained 23.3 wt.% HQ, 3.0 wt.% MAHQ, 0.0 wt.% DAHQ, 21.7 wt.% acetic acid, 41.8 wt.% ethyl acetate (an increase of 6.9 grams) and 5.5 wt.% ethanol (a decrease of 3.6 grams). These results show that the normalized conversion of MAHQ to HQ was 91.5% and that the normalized efficiency to HQ was 100% based on product accountability of 105.7%.

The procedures of Examples 1 to 10 can also be used to prepare for example, 3-phenylcatechol from 2-hydroxy-5-phenyl-acetophenone; phenylhydroquinone from 4-hydroxy-3-phenyl-acetophenone; methylhydroquinone from 4-hydroxy-3-methyl-acetophenone; catechol from 2-hydroxyacetophenone (2-HAP); 2,6-dihydroxynaphthalene (2,6-DHN) from 6-acetoxy-2-acetonaphthone; and 4,4'-biphenol from 4(4'-hydroxyphenyl) acetophenone.

The alcoholysis procedure illustrated in Examples 9 and 10 may also be carried out using methanol, propanol or n-butanol to obtain methyl, propyl or n-butyl acetate respectively as a co-product with HQ.

**Claims**

1. A process comprising subjecting an aromatic ketone containing a hydroxy radical on the aromatic ring, to a Baeyer-Villiger oxidation using peracetic acid as oxidant to produce a reaction product comprising a carboxylate ester of an aromatic diol, and contacting a composition comprising a major proportion of said carboxylate ester with an alcohol or phenol at an elevated temperature and under acidic conditions for a period of time to transesterify said carboxylate ester to the free aromatic diol and a newly-formed ester of said alcohol or phenol with the carboxylic acid moiety of said carboxylate ester, said peracetic acid being employed such that the reaction mass of the Baeyer-Villiger oxidation contains no more than 0.1 wt.% of sulfuric acid based on the weight of peracetic acid initially added.

2. The process of claim 2 wherein said alcohol is an alkanol containing 1 to 4 carbon atoms.

3. The process of claim 2 wherein said aromatic ketone is 4-hydroxyacetophenone, said monocarboxylate ester is the monoacetate ester of hydroquinone, said alcohol is ethanol, said aromatic diol is hydroquinone and said newly-formed ester is ethyl acetate.

4. The process of any of claims 1—3 wherein the reaction mass of the Baeyer-Villiger oxidation also contains no more than 5 wt.% of water based on the weight of peracetic acid initially added.

**Patentansprüche**

1. Verfahren dadurch gekennzeichnet, daß ein aromatisches Keton, enthaltend einen Hydroxylrest am aromatischen Ring, unter Verwendung von Peressigsäure als Oxydationsmittel zwecks Bildung eines Reaktionsproduktes, bestehend aus einem Carboxylatester eines aromatischen Diols, einer Baeyer-Villiger Oxydation unterworfen wird und eine Zusammensetzung, bestehend zu einem größeren Teil aus dem Carboxylester mit einem Alkohol oder Phenol bei einer erhöhten Temperatur und unter sauren Bedingungen für eine Zeitdauer in Berührung gebracht wird, um die Umesterung des Carboxylatesters zum freien aromatischen Diol und einem neue gebildeten Ester des Alkohols oder Phenols mit dem Carbonsäureteil des Carboxylatesters zu bewirken, wobei die Peressigsäure in der Weise eingesetzt wird, daß dieas Reaktionsmasse der Baeyer-Villiger Oxydation nicht mehr als 0.1 Gew.-% Schewefelsäure, bezogen auf das Gewicht der anfänglich zugesetzten Peressigsäure, enthält.

2. Verfahren nach Anspruch 1, dadurch gekennzeichnet, daß der Alkohol ein Alkanol enthaltend ein 1 bis 4 Kohlenstoffatome ist.

3. Verfahren nach Anspruch 2, dadurch gekennzeichnet, daß das aromatische Keton ein 4-Hydroxyacetophenon, der Monocarboxylatester der Monoacetatester von Hydrochinon, der Alkohol Ethanol, das aromatische Diol Hydrochinon und der neu gebildete Ester Ethylacetat ist.

4. Verfahren nach einem der Ansprüche 1 bis 3, dadurch gekennzeichnet, daß die Reaktionsmasse der Baeyer-Villiger Oxydation auch nicht mehr als 5 Gew.-% Wasser, bezogen auf das Gewicht der anfänglich zugesetzten Peressigsäure, enthält.

**Revendications**

1. Procédé consistant à soumettre une cétone aromatique contenant un radical hydroxy sur le noyau aromatique, à une oxydation de Baeyer-Villiger en utilisant l'acide peracétique comme oxydant pour produire un produit réactionnel comprenant un ester de carboxylate d'un diol aromatique, et à mettre une composition comprenant une proportion majeure dudit ester de carboxylate en contact avec un alcool ou phénol à une température élevée et an conditions acides pendant une période de temps pour transestérifier ledit ester de carboxylate en diol aromatique libre et un ester nouvellement formé dudit alcool ou phénol avec le fragment d'acide carboxylique dudit ester de carboxylate, ledit acide peracétique étant employé de manière que la masse réactionnelle de l'oxydation de Baeyer-Villiger ne contienne pas plus de 0,1% en poids d'acide sulfurique en se basant sur le poids de l'acide peracétique initialement ajouté.

2. Procédé de la revendication 2 où ledit alcool est un alcanol contenant 1 à 4 atomes de carbone.

3. Procédé de la revendication 2 où ladite cétone aromatique est la 4-hydroxyacétophénone, ledit ester de monocarboxylate est l'ester de monoacétate d'hydroquinone, le dialcool est l'éthanol, ledit diol aromatique est l'hydroquinone et ledit ester nouvellement formé est l'acétate d'éthyle.

4. Procédé selon l'une des revendications 1—3 où la masse réactionnelle de l'oxydation de Baeyer-Villiger ne contient également pas plus de 5% en poids d'eau en se basant sur le poids de l'acide peracétique initialement ajouté.